# EUROPEAN PATENT SPECIFICATION

(11) **EP 2 213 326 B1**
(45) Date of publication and mention of the grant of the patent: **27.01.2016**
(21) Application number: 08800731.5
(22) Date of filing: 01.09.2008
(51) Int. Cl.: A61K 41/00, A61M 37/00, A61M 35/00, A61N 7/00

(54) **ULTRASONIC MEDICINE PASTE**
MEDIZINISCHE ULTRASCHALLPASTE
PÂTE DE MÉDICAMENT ULTRASONORE

(30) Priority: 13.11.2007 CN 200710188165
(43) Date of publication of application: 04.08.2010
(73) Proprietor: Chongqing Ronghai Engineering Research Center of Ultrasonic Medicine Co., Ltd., Renhe, Yubei District Chongqing, 401121 (CN)
(72) Inventor: WANG, Qing, Chongqing 400039 (CN)
(74) Representative: Pulieri, Gianluca Antonio
(86) International application number: PCT/CN2008/072219
(87) International publication number: WO 2009/062421

(56) References cited:
- EP-A1- 0 495 531
- WO-A2-03/061726
- WO-A2-2004/060447
- CN-A- 1 671 326
- CN-A- 101 143 242
- JP-A- 3 198 871
- JP-A- 03 063 071
- US-A- 5 007 438
- US-A- 5 617 851
- US-A1- 2005 182 389
- US-B1- 6 322 532

## Description

The present application claims the priority to China Patent Application No. 200710188165.6 entitled "DISPOSABLE ULTRASONIC MEDECINE PATCH" filed in China on November 13, 2007, the entire content of which is hereby incorporated by reference.

### FIELD OF THE INVENTION

The present invention relates to an ultrasonic medical device, and in particular, to a device for the transdermic delivery of medicaments into organisms by utilizing ultrasonic waves.

### BACKGROUND OF THE INVENTION

At present, there are three major administration routes for existing medicine therapies, i.e. injection, oral administration, and transdermal administration Injections need to be performed intravenously or intramuscularly, and suffers from infections that may be caused. Circulated in blood, medicaments act on the suffering site in a relatively short period, and a major part of them is eliminated, rendering a substantial waste. Orally administrated medicines have a reduced efficacy after digestion and absorption by the digestive system. Also, most of medicaments are bitter in taste, which poses difficulties for the medicaments to be ingested by patients. Therefore, it has become a promising area of medicine to apply medicaments transdermally into diseased sites. Conventional topical application applies medicines directly onto affected sites by dressing or bandaging. Due to poor permeability of skins and muscle tissues, the therapeutic period is prolonged, which causes much inconvenience to patients. With the increasing development of medical science, medical devices for delivering of medicines into the body of a subject via electronic and ultrasonic means are developed, and they effectively enhance the absorption of medicaments and the therapeutic effect. Compared with electronic devices, ultrasonic devices have been more frequently utilized for being non-invasive and non-radiative. In the prior art, ultrasonic devices for transdermally delivering a medicine mainly ulitize ultrasonic atomization, heat-introduction, oscillation-introduction and the like. China Patent Application No. 90103647.1 discloses an ultrasonic device for permeating medicine solutions, in which a fabric or cotton paper soaked with the medicine solution is applied to affected sites, then an ultrasonic probe is placed thereon and moved back and forth, thereby permeating the medicine solution into tissues via High-frequency vibration, to achieve the therapeutic effect. China Patent Application No. 03802476.4 discloses an ultrasonic transdermal device, an ultrasonic transdermal auxiliary product and a method for ultrasonic transdermal delivery. The application discloses a device and method for safely and effectively permeating medicines to the sites to be treated through skin, muscle and fat. Furthermore, ultrasonic waves with different frequency are used for permeating medicines into different sites. However, the prior art disclosed techniques only employ ultrasonic wave as a means for improving medicine permeation, and ultrasonic waves and medicines are not considered as an entity, not to mention the integration or miniaturization thereof.

EP 0495531 discloses an endermic application kit for external medicines, which comprises a drug-containing layer as provided near an ultrasonic oscillator. The kit includes a cylindrical fixed-type or portable-type and a flat regular-type or adhesive-type, and the adhesive-type may be flexible and elastic. The drug absorption is ensured by the action of the ultrasonic waves from the oscillator and the drug release can be controlled by varying the ultrasonic wave output from the oscillator.

US 5617851 discloses a noninvasive method and apparatus for withdrawing fluid from an organism and determining the concentration of a substance in the fluid is disclosed. The noninvasive apparatus comprises a substance concentration sensor that includes an extraction transducer and a substance sensing transducer. The extraction transducer includes a container; a planar piezoelectric (ultrasonic) transducer; a focusing lens; and a backing layer. The container includes a flange that is attached to the skin of an organism by an adhesive film seal. The focusing lens is a plano-concave shape such that the concave side faces the skin of the organism. Thus, the focusing lens defines a cavity. Located in the cavity is the substance sensing transducer. Positioned above the focusing lens is the ultrasonic transducer. A pulse generator is connected to and drives the ultrasonic transducer. A test data processor is connected to the substance sensing transducer. Ultrasonic transducer pulses are focused by the focusing lens into the organism, traumatizing the skin underlying the cavity. The trauma causes the dermal-epidermal junction membrane and the capillary endothelial joints to open and allow fluid to be drawn from the cavity. The substance to be detected in the fluid is sensed by the substance sensing transducer causing the output of the transducer to change. The output changes are analyzed by the test data processor.

US 6322532 discloses an ultrasonic transducer that operates in flexure mode provides a highly efficient and compact sonophoresis device. Such a device is particularly useful for efficiently enhancing permeation of a substance through a membrane, such as dermal and mucosal membranes for purposes of transdermal/transmucosal drug delivery and/or body fluid monitoring.

To sum up, the ultrasonic transdermal delivery devices in the prior art suffer from disadvantages such as the complexity in structure, being inconvenient to be used by patients on their own, medicines being discreted with the ultrasonic system, which tends to cause off-target and in turn damage to surrounding tissues, and using the same probe to treat several patients, which tends to cause cross-contamination, or the like. The integrative ultrasonic medicine patch according to the present invention completely solves the above problems. Besides, it presents a low-cost and is suitable to be produced and commercialized in a large scale.

### SUMMARY OF THE INVENTION

The present invention overcomes the above disadvantages and provides an ultrasonic medicine patch as defined in claim 1. The medicine patch is simple in structure, and is easy to use and prepare.

The technical solution used to solve the technical problems according to the present invention is as follows:
the ultrasonic medicine patch comprises an adhesive layer as the base layer, a medicine layer immediately adjacent to the adhesive layer, an ultrasonic transducer generating ultrasonic signals, a driving unit providing electric signals and driving the ultrasonic transducer to generate ultrasonic signals, and a coating layer that covers the exterior surfaces and forms the patch into one entity.

The adhesive layer is made of flexible materials with good permeability, such as perforated cloth, either of the surfaces of which is coated with an adhesive for avoiding cross contamination. The surface opposite to the one coated with the adhesive has two surface regions, that is, the first region and the second region.

The surface of the adhesive layer, on which an adhesive is coated, is further attached with a release film. During treatment, the release film can be peeled off, and the patch can be attached to the sites to be treated.

The medicine layer, made of an adsorbing material sprayed with or soaked in the medicines, is fixed on the first region through pressing and adhering and the like. Immediately adjacent to the medicine layer is the ultrasonic wave emitting surface of the ultrasonic transducer.

The driving unit is located on the back of the ultrasonic transducer, and comprises an ultrasonic driving circuit and a power supply, integrated or not.

The power supply is integrated in the patch: it is disposed in the same layer as the ultrasonic driving circuit, and on the back of the ultrasonic transducer. The power supply is electrically connected with the ultrasonic transducer and the ultrasonic driving circuit, being switched off by an on/off film, one end of which being exposed to the outside of the patch. A part of the coating layer integratively covers the medicine layer, the ultrasonic transducer and the driving unit, and fixes them on the first region of the adhesive layer, while the other part is combined with the second region of the adhesive layer, to form a freely flexible bonding edge. In operation, the on/off film is withdrawn, and the power supply is switched on to operate the patch.

In an example not forming part of the invention, the power supply is placed outside the coating layer, it comprises an external battery box, a battery, a power lead, a plug and the fixed socket disposed on the patch. The battery is placed in the battery box, and connected to the plug via the power lead. The fixed socket on the patch is electrically connected with the ultrasonic driving circuit and the ultrasonic transducer. A part of the coating layer integratively covers the medicine layer, the ultrasonic transducer and the driving unit, and fixes them on the first region of the adhesive layer, while the other part is combined with the second region of the adhesive layer to form a freely flexible bonding edge. In operation, the plug is inserted in the fixed socket, and the power supply is switched on to operate the patch.

Corresponding to the ultrasonic patch of the invention, a method for manufacturing the patch is also provided, comprising:
coating an adhesive on an adhesive layer, the surface opposite to the surface coated with the adhesive having two surface regions, the first region and the second region, the adhesive layer being the lowest layer of the present patch, and being made of a flexible material having good permeability;
fixing a medicine layer, an ultrasonic transducer, a driving unit on the first region;
coating the second region with an adhesive and combining it with the coating layer;
a strongly adsorbing material is sprayed with or soaked in a medicine, to form the medicine layer;

Fixing the medicine layer on the first region of the adhesive layer, with the ultrasonic wave emitting surface immediately attached to the ultrasonic transducer;
placing an on/off film to keep the power supply off, one end of the on/off film being exposed to the outside of the patch, to facilitate being withdrawn in operation, to switch on the power supply;
integratively covering the medicine layer, the ultrasonic transducer and the driving unit and fixing them on the first region of the adhesive layer with a part of the coating layer, while the other part of the coating layer is combined with the second region of the adhesive layer to form a freely flexible bonding edge, so that the patch can fit on an unflat skin surface of a human body.

The present invention has the following advantages: the present invention skillfully incorporates the ultrasonic system into the medicine patch, greatly improving the utility of the patch, while keeping the patch light, convenient and disposable, so that the pates can be put into clinical use sooner and better. The present invention is simple in structure, easy to manufacture, and convenient to use, and can avoid adverse outcomes caused by injections, such as infection, pain, and fear. The present structure enables the ultrasonic patch to be used in the same way as conventional patchs, thus addresses the key problem that hinders the ultrasonic medicine permeating technologies from being wiled used in clinic.

### BRIEF DESCRIPTION OF THE DRAWINGS

Fig. 1 is the schematic view of the present invention.
Fig. 2 is the sectional view of example 1 which describes an ultrasonic medicine patch according to the present invention.
Fig. 3 is the sectional view of example 2 which does not from part of the invention.
Fig. 4 is the schematic view of external power supply of example 2.
Fig. 5 is the schematic view of a contour of the bonding edge of the present patch.
Fig. 6 is the schematic view of another contour of the bonding edge of the present patch.
Reference signs: 1. adhesive layer; 2. medicine layer; 3. ultrasonic transducer; 4. driving unit; 41. power supply; 42. ultrasonic driving circuit; 5. coating layer; 7. on/off film; 11. the first region; 12. the second region; 60. battery box; 61. socket; 62. plug; 63. power lead; 64. battery.

### DETAILED DESCRIPTION OF EMBODIMENTS

The structures of the present invention and the effects can be achieved will be further described below in detail in connection with the drawings.

### Example 1

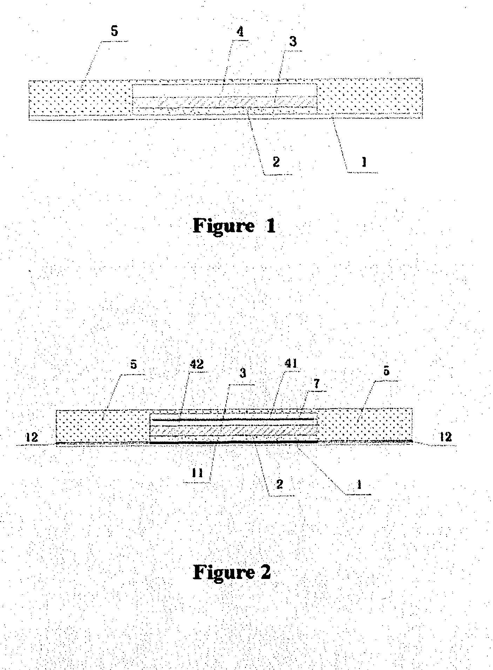

As shown in fig. 1, the ultrasonic medicine patch according to the present invention comprises an adhesive layer 1, a medicine layer 2, a ultrasonic transducer 3, a driving unit 4, and a coating layer 5. The medicine layer 2, ultrasonic transducer 3 and driving unit 4 are sequentially laminated from bottom to top, with coating layer 5 covering the three layers. Adhesive layer 1 is the base layer.

As shown in fig. 2, adhesive layer 1 is the lowest layer of the present patch, and is made of a flexible material having good permeability. In this example, the adhesive layer 1 is made of perforated cloth, either of the surfaces of which is coated with an adhesive for one-time attachment. The other surface of the adhesive layer has two regions, a first region 11 which is preferably located at the center of the cloth and used to fix medicine layer 2, ultrasonic transducer 3 and driving unit 4 thereon; and a second region 12 which is preferably located on the peripheral of the cloth, and can be coated with an adhesive for adhering to the coating layer. The patch can also be made in a manner that the first region 11 is not located at the center, and the second region 12 is asymmetrical, facilitating the treatment of specific sites.

The medicine layer 2 is made of a strongly adsorbing material, which has been sprayed with a medicine thereon, or has been soaked with a medicines. In this example, nonwoven fabrics that has been soaked with a medicine solution are preferred. The medicine layer is fixed on the first region 11 of the adhesive layer 1. Immediately attached to the medicine layer is the ultrasonic wave emitting surface of the ultrasonic transducer 3.

In the example, the driving unit 4 comprises a power supply 41 and an ultrasonic driving circuit 42, which is located on the back surface of the ultrasonic transducer 3. The power supply 41 is electronically connected respectively to the ultrasonic driving circuit 42 and the ultrasonic transducer 3, which is preferably a battery. An on/off film 7 is used to keep the power supply in opening state, and one of its ends is exposed to the outside of the patch, facilitating the withdrawing thereof in operation, so as to switch on the power supply.

A part of the coating layer 5 integrally covers the medicine layer 2, the ultrasonic transducer 3 and the driving unit 4, and fixes them on the first region 11 of the adhesive layer 1, while the other part is combined with the second region 12 of the adhesive layer 1 to form a freely flexible bonding edge, so that the patch can fit an unflat skin surface of a patient.

Furthermore, this example comprises a disposable release film, which is attached on the surface of the adhesive layer being coated with the adhesive, and can be disposed upon use.

When using the medicine patch of the present invention for treatment, the release film is firstly peeled off, and the patch is bonded via the adhesive layer 1 onto the skin surface of the sites requiring treatment. Subsequently, the on/off film 7 is withdrawn by pulling its end exposed to the outside, to switch on the power supply, so that the ultrasonic transducer 3, driven by the ultrasonic driving circuit 42, emits ultrasonic wave toward the medicine layer 2. Thus the solution of the medicine is permeated through the adhesive layer 1 having a good permeability, the skin of the body, the tissues and the like and into the affected sites, where the medicine acts and achieve the treatment.

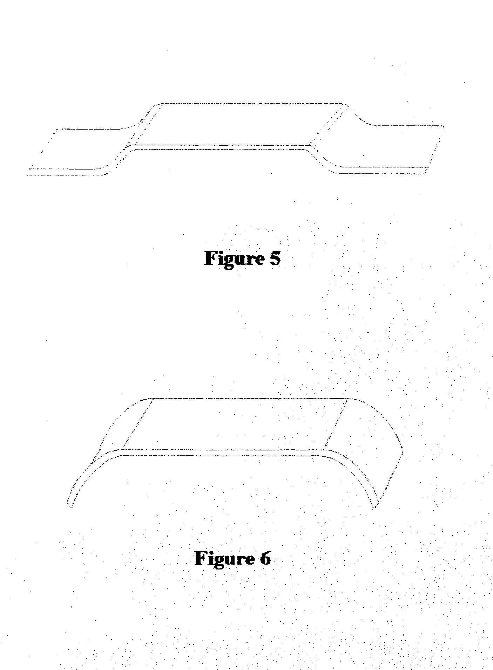

Figs. 5 and 6 are schematic views of bended bonding edges formed by combining the coating layer 5 and the second region 12 on the adhesive layer 1. However, the contour of the bonding edges is not limited thereto. Since the present medicine patch is small in size and simple, it can be easily attached on affected skins during treatment, and can be peeled off after treatment.

### Example 2

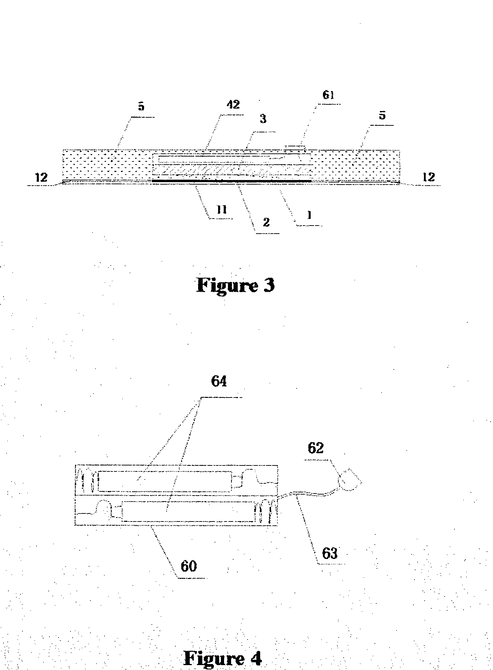

As shown in figs. 3 and 4, in order to further minimize the medicine patch, this example differs from example 1 in that the power supply 41 in the driving unit 4 is not integrated. The external power supply 41 comprises a battery box 60, a battery 64, a power lead 63, a plug 62, and fixed socket 61 disposed on the patch. The battery 64 is placed in the battery box 60 and is connected to the plug 62 via the power lead 63 to form an electric pathway. The fixed socket 61 is electrically connected to the driving unit 42 and the ultrasonic transducer 3. In this example, a part of the coating layer 5 covers the medicine layer 2, the ultrasonic transducer 3 and the ultrasonic driving unit 42 and fixes them on the first region 11 of the adhesive layer 1, while the other part is combined with the second region 12 of the adhesive layer 1 to form a freely flexible bonding edge. When using the patch, the plug 63 is inserted into the socket 64 to switch on the power supply, and the patch begins to work.

It will be appreciated by those skilled in the art that the ultrasonic transducer can be selected from those having different working frequencies, and the number of the ultrasonic transducers is not limited to only one. In stead, an appropriate number of ultrasonic transducers can be selected as needed, so that the patch can work under different frequencies. The ultrasonic transducers having different frequencies can operate alternately or simultaneously.

The means to switch on/off of the power supply of the ultrasonic transducer is not limited to the on/off film. Other known means in the art can also be used without departing from the scope of the present invention.

Corresponding to the example of the present invention (example 1) a method for manufacturing an ultrasonic patch is described as comprising the steps of:
300. coating an adhesive onto one surface of an adhesive layer, the other surface having two surface regions, the first region 11 and the second region 12. The adhesive layer 1 is the lowest layer of the present patch, and is made of a flexible material having good permeability. In this example, adhesive layer 1 is made of perforated cloth, with either of its surfaces being coated with an adhesive for one-time attachment;
301. fixing the medicine layer 2, ultrasonic transducer 3 and driving unit 4 on the first region11, the first region 11 preferably being located at the center of the patch;
302. coating the second region with an adhesive and combining it with the coating layer; the second region 12 preferably being a peripheral region, and can be coated with an adhesive for combining with the coating layer. The patch can also be made so that the first region 11 is not located at the center, and the second region 12 is asymmetrical, facilitating the treatment on sites with specific shapes;
303. making a medicine layer 2 by applying to a strongly adsorbing material a medicine, through spraying or soaking. Nonwoven fabrics soaked with a medicine solution are preferred;
304. fixing the medicine layer onto the first region 11 of the adhesive layer 1. Immediately attached to the medicine layer is the ultrasonic wave emitting surface of the ultrasonic transducer 3.

In the example of the present invention (example 1), driving unit 4 comprises a power supply 41 and an ultrasonic driving circuit 42, which is located on the back surface of the ultrasonic transducer 3. The power supply 41 is electronically connected to the ultrasonic driving circuit 42 and the ultrasonic transducer 3, and is preferably a battery. An on/off film 7 is used to keep the power supply in opening state, and one of its ends is exposed to the outside of the patch, facilitating the withdrawing thereof in operation, so as to switch on the power supply;
305. integrally covering the medicine layer 2, the ultrasonic transducer 3 and the driving unit 4 with a part of the coating layer 5, and fixes them on the first region 11 of the adhesive layer 1, while the other part of the coating layer 5 is combined with the second region 12 of the adhesive layer 1 to form a freely flexible bonding edge, so that the patch can fit an unflat skin surface of a patient.

Steps 300-304 describe a method for manufacturing an ultrasonic patch according to example 2 which does not form part of the invention.

Furthermore, this example comprises a release film, which is attached on the surface of the adhesive layer that is coated with adhesive, and can be disposed upon use.

When using the medicine patch of the present invention for treatment, the release film is firstly peeled off, and the patch is bonded via the adhesive layer 1 onto the skin surface of the sites requiring treatment. Subsequently, the on/off film 7 is withdrawn by pulling its end exposed to the outside, to switch on the power supply, so that the ultrasonic transducer 3, driven by the ultrasonic driving circuit 42, emits ultrasonic wave toward the medicine layer 2. Thus the solution of the medicine is permeated through the adhesive layer 1 having a good permeability, the skin of the body, the tissues and the like and into the affected sites, where the medicine acts and achieve the treatment.

As shown in figs. 3 and 4, in order to further minimize the medicine patch, the power supply 41 in the driving unit 4 is not integrated. The external power supply 41 comprises a battery box 60, a battery 64, a power lead 63, a plug 62, and fixed socket 61 disposed on the patch. The battery 64 is placed in the battery box 60 and is connected to the plug 62 via the power lead 63 to form an electric pathway. The fixed socket 61 is electrically connected to the driving unit 42 and the ultrasonic transducer 3. In this example, a part of the coating layer 5 covers the medicine layer 2, the ultrasonic transducer 3 and the ultrasonic driving unit 42 and fixes them on the first region 11 of the adhesive layer 1, while the other part is combined with the second region 12 of the adhesive layer 1 to form a freely flexible bonding edge. When using the patch, the plug 63 is inserted into the socket 64 to switch on the power supply, and the patch begins to work.

It will be appreciated by those skilled in the art that, various medicines can be soaked into the patch and permeated into organisms, depending on the therapeutic needs and the specific conditions (such as pharyngitis, osteoarticular diseases, painful muscle, skin diseases, acupuncture treatments in traditional Chinese medicine, and the like) or health care needs (such as cosmetology, whitening, wrinkle removing and weight controlling). The ultrasonic medicine patchs disclosed in example 1 of the present invention and in example 2 not forming part of the invention are characterized by miniaturization and integration, in which the size of the ultrasonic transducer and the driving unit is comparable to that of a round button cell, and the thickness thereof is only about 3-4 times that of the cell. The volume thereof is only about 1/(100∼1000) of the ultrasonic medicine permeating device in the prior art, while the intensity and duration of the ultrasonic waves emitted can sufficiently meet the needs for medicine permeation. When the technology according to the present invention is applied to deliver analgesics to some of the patients suffering from painful muscle and bone fracture, an obvious analgesic effect can be achieved within only 10-20 minutes, 3-5 times faster than simply applying the medicines.

## Claims

1. An ultrasonic medicine patch comprising, from bottom to top, an adhesive layer (1) as the base layer, a laminate sequentially containing a medicine layer (2) immediately adjacent to the adhesive layer (1), an ultrasonic transducer (3) generating an ultrasonic signal, a driving unit (4) providing an electric signal and driving the ultrasonic transducer (3) to generate the ultrasonic signal, and a coating layer (5) that covers the exterior surface of the patch and covers the medicine layer (2), the ultrasonic transducer (3) and the driving unit (4), wherein the driving unit (4) comprises an ultrasonic driving circuit (42) electrically connected with a power supply (41) placed and arranged inside the coating layer (5);
said patch being **characterized in that** the power supply (41) is integrated with the ultrasonic driving circuit (42), and disposed on the back of the ultrasonic transducer (3); and **in that** the power supply (41) is electrically connected to the ultrasonic transducer (3) and the ultrasonic driving circuit (42), the power supply (41) being switched off by an on/off film (7), wherein one end of the film (7) is exposed to the outside of the coating layer (5) for being withdrawn in operation, so as to switch on the power supply (41) to operate the patch.

2. The ultrasonic medicine patch according to claim 1, wherein the adhesive layer (1) is made of a flexible material having a good permeability.

3. The ultrasonic medicine patch according to claims 1 or 2, wherein the adhesive layer (1) is made of perforated cloth.

4. The ultrasonic medicine patch according to claims 1 or 2, wherein a release film is attached to the surface of the adhesive layer (1), on which an adhesive is coated.

5. The ultrasonic medicine patch according to claim 1, wherein the medicine layer (2) is made of an adsorbing material, onto which a medicine is sprayed, or which has been soaked with a medicine.

6. The ultrasonic medicine patch according to claim 2, wherein one surface of the flexible material is coated with an adhesive; and the opposite surface comprise two surface regions, the first region and the second region.

7. The ultrasonic medicine patch according to claim 1, wherein the coating layer (5) is made of a flexible material, a part of which is used for covering the medicine layer (2), the ultrasonic transducer (3) and the driving unit (4), while the other part is combined with the second region of the adhesive layer (1) to form a freely flexible bonding edge.

8. A method for manufacturing an ultrasonic medicine patch, comprising the steps of:
coating one surface of an adhesive layer (1) with an adhesive, wherein the opposite surface of the adhesive layer (1) comprises two surface regions, the first region (11) and the second region (12), the adhesive layer (1) being the lowest layer of the patch, and being made of a flexible material having good permeability;
fixing a medicine layer (2), an ultrasonic transducer (3) and a driving unit (4) on the first region (11), the driving unit (4) comprising a power supply (41) and an ultrasonic driving circuit (42), which in located in the back surface of the ultrasonic transducer (3), the power supply (41) being electronically connected to the ultrasonic driving circuit (42) and the ultrasonic transducer (3);
coating the second region (12) with an adhesive and combining it with the coating layer (5);
spraying a medicine onto an adsorbing material, or soaking an adsorbing material with a medicine, to form a medicine layer (2);
fixing the medicine layer (2) on the first region (11), with the ultrasonic wave emitting surface of the ultrasonic transducer (3) immediately attached to the medicine layer (2);
integrally covering the medicine layer (2), the ultrasonic transducer (3) and the driving unit (4) and fixing them on the first region (11) of the adhesive layer (1) with a part of the coating layer (5), and combining the other part with the second region (12) of the adhesive layer (5) to form a freely flexible bonding edge, so that the patch can fit an unflat skin surface of a human body;
placing an on/off film (7) to keep the power supply (41) off, wherein one end of the on/off film (7) is exposed to the outside of the patch for being withdrawn in operation, to switch on the power supply (41).

9. The method according to claim 8, wherein the first region (11) is not located at the center of the adhesive layer (1), and the second region (12) is an asymmetrical peripheral.

## Patentansprüche

1. Medizinisches Ultraschallpatch umfassend, von unten nach oben, eine Klebstoffschicht (1) als die Grundschicht, einen Schichtstoff sequenziell beinhaltend eine Arzneimittelschicht (2) direkt angrenzend an die Klebstoffschicht (1), einen Ultraschall-Transducer (3), der ein Ultraschallsignal erzeugt, eine Antriebseinheit (4), die ein elektrisches Signal bereitstellt und den Ultraschall-Transducer (3) antreibt, das Ultraschallsignal zu erzeugen, und eine Deckschicht (5), die die äußere Fläche des Patches bedeckt sowie die Arzneimittelschicht (2), den Ultraschall-Transducer (3) und die Antriebseinheit (4) bedeckt, wobei die Antriebseinheit (4) eine Ultraschall-Treiberschaltung (42) umfasst, die elektrisch mit einer Leistungsversorgung (41) verbunden und innerhalb der Deckschicht (5) platziert und angeordnet ist;
wobei das Patch **dadurch gekennzeichnet ist, dass** die Leistungsversorgung (41) mit der Ultraschall-Treiberschaltung (42) integriert ist, und auf der Rückseite des Ultraschall-Transducers (3) angebracht ist; und dass die Leistungsversorgung (41) elektrisch mit dem Ultraschall-Transducer (3) und der Ultraschall-Treiberschaltung (42) verbunden ist, wobei die Leistungsversorgung (41) durch einen An-/Aus-Film (7) ausgeschaltet wird, wobei ein Ende des Films (7) zur Außenseite der Deckschicht (5) hin freigelegt ist um bei Betrieb zurückgezogen zu werden, zum Einschalten der Leistungsversorgung (41) zur Bedienung des Patches.

2. Medizinisches Ultraschallpatch nach Anspruch 1, wobei die Klebstoffschicht (1) aus einem flexiblen Material mit einer guten Permeabilität hergestellt ist.

3. Medizinisches Ultraschallpatch nach Anspruch 1 oder 2, wobei die Klebstoffschicht (1) aus einem perforierten Stoff hergestellt ist.

4. Medizinisches Ultraschallpatch nach Anspruch 1 oder 2, wobei ein Freigabefilm an der Fläche der Klebstoffschicht (1) angebracht ist, auf welcher ein Klebstoff aufgetragen ist.

5. Medizinisches Ultraschallpatch nach Anspruch 1, wobei die Arzneimittelschicht (2) aus einem adsorbierenden Material hergestellt ist, auf das ein Arzneimittel gesprüht ist oder das mit einem Arzneimittel getränkt wurde.

6. Medizinisches Ultraschallpatch nach Anspruch 2, wobei eine Fläche des flexiblen Materials mit einem Klebstoff beschichtet ist; und die gegenüberliegende Fläche zwei Flächenbereiche umfasst, den ersten Bereich und den zweiten Bereich.

7. Medizinisches Ultraschallpatch nach Anspruch 1, wobei die Deckschicht (5) aus einem flexiblen Material hergestellt ist, von dem ein Teil dafür benutzt wird, die Arzneimittelschicht (2), den Ultraschall-Transducer (3) und die Antriebseinheit (4) zu bedecken, während der andere Teil mit dem zweiten Bereich der Klebstoffschicht (1) verbunden ist um eine frei flexible Klebekante zu bilden.

8. Verfahren zur Herstellung eines medizinischen Ultraschallpatches, umfassend die folgenden Schritte:
Beschichten einer Fläche einer Klebstoffschicht (1) mit einem Klebstoff, wobei die der Klebstoffschicht (1) gegenüberliegende Fläche zwei Flächenbereiche umfasst, den ersten Bereich (11) und den zweiten Bereich (12), wobei die Klebstoffschicht (1) die unterste Schicht des Patches ist und aus einem flexiblen Material mit einer guten Permeabilität hergestellt ist;
Fixierung einer Arzneimittelschicht (2), eines Ultraschall-Transducers (3) und einer Antriebseinheit (4) auf dem ersten Bereich (11), die Antriebseinheit (4) umfassend eine Leistungsversorgung (41) und eine Ultraschall-Treiberschaltung (42), die sich an der hinteren Fläche des Ultraschall-Transducers (3) befindet, wobei die Leistungsversorgung (41) elektronisch mit der Ultraschall-Treiberschaltung (42) und dem Ultraschall-Transducer (3) verbunden ist;
Auftragen eines Klebstoffs auf den zweiten Bereich (12) und Verbinden mit der Deckschicht (5);
Sprühen eines Arzneimittels auf ein adsorbierendes Material, oder Tränken eines adsorbierenden Materials mit einem Arzneimittel zur Bildung einer Arzneimittelschicht (2);
Fixieren der Arzneimittelschicht (2) auf dem ersten Bereich (11), wobei die ultraschallwellenemittierend Fläche des Ultraschall-Transducers (3) unmittelbar an der Arzneimittelschicht (2) angebracht ist;
integrales Bedecken der Arzneimittelschicht (2), des Ultraschall-Transducers (3) und der Antriebseinheit (4) und Fixieren dieser auf dem ersten Bereich (11) der Klebstoffschicht (1) mit einem Teil der Deckschicht (5), sowie Kombinieren des anderen Teils mit dem zweiten Bereich (12) der Klebstoffschicht (5) um eine frei flexible Klebekante zu bilden, so dass das Patch einer unebenen Hautfläche des menschlichen Körpers passen kann;
Platzieren eines An-/Aus-Films (7) um die Leistungsversorgung (41) abgeschaltet zu lassen, wobei ein Ende des An-/Aus-Films (7) zur Außenseite des Patches freigelegt ist um bei Betrieb zurückgezogen zu werden, um die Leistungsversorgung (41) einzuschalten.

9. Das Verfahren nach Anspruch 8, wobei der erste Bereich (11) sich nicht in der Mitte der Klebstoffschicht (1) befindet sowie der zweite Bereich (12) asymmetrisch peripher ist.

## Revendications

1. Timbre transdermique médicamenteux à ultrasons comprenant, de bas en haut, une couche adhésive (1) constituant la couche de base, un stratifié contenant successivement une couche médicamenteuse (2) immédiatement adjacente à la couche adhésive (1), un transducteur à ultrasons (3) générant un signal ultrasonore, une unité d'excitation (4) fournissant un signal électrique et excitant le transducteur à ultrasons (3) de façon à générer le signal ultrasonore, et une couche de couverture (5) qui couvre la surface extérieure du timbre transdermique et recouvre la couche médicamenteuse (2), le transducteur à ultrasons (3) et l'unité d'excitation (4), dans lequel l'unité d'excitation (4) comprend un circuit excitateur d'ultrasons (42) connecté électriquement à une alimentation électrique (41) placée et disposée à l'intérieur de la couche de couverture (5),
ledit timbre transdermique étant **caractérisé en ce que** l'alimentation électrique (41) est intégrée avec le circuit excitateur d'ultrasons (42) et disposé sur l'arrière du transducteur à ultrasons (3), et **en ce que** l'alimentation électrique (41) est connectée électriquement au transducteur à ultrasons (3) et au circuit excitateur d'ultrasons (42), l'alimentation électrique (41) étant désactivée par un interrupteur à film (7), une extrémité du film (7) étant exposée à l'extérieur de la couche de couverture (5) afin d'être retirée, en fonctionnement, de façon à activer l'alimentation électrique (41) pour faire fonctionner le timbre transdermique.

2. Timbre transdermique médicamenteux à ultrasons selon la revendication 1, dans lequel la couche adhésive (1) est faite d'une matière flexible ayant une bonne perméabilité.

3. Timbre transdermique médicamenteux à ultrasons selon la revendication 1 ou 2, dans lequel la couche adhésive (1) est faite de toile perforée.

4. Timbre transdermique médicamenteux à ultrasons selon la revendication 1 ou 2, dans lequel un film pelable est fixé à la surface de la couche adhésive (1), qui est enduite d'un adhésif.

5. Timbre transdermique médicamenteux à ultrasons selon la revendication 1, dans lequel la couche médicamenteuse (2) est faite d'une matière absorbante sur laquelle un médicament est pulvérisé ou qui a été imprégnée d'un médicament.

6. Timbre transdermique médicamenteux à ultrasons selon la revendication 2, dans lequel une surface de la matière flexible est enduite d'un adhésif et la surface opposée comporte deux régions de surface, la première région et la deuxième région.

7. Timbre transdermique médicamenteux à ultrasons selon la revendication 1, dans lequel la couche de couverture (5) est faite d'une matière flexible dont une partie est utilisée pour couvrir la couche médicamenteuse (2), le transducteur à ultrasons (3) et l'unité d'excitation (4), tandis que l'autre partie est combinée avec la deuxième région de la couche adhésive (1) pour former un bord de collage flexible à volonté.

8. Procédé de fabrication d'un timbre transdermique médicamenteux à ultrasons, comprenant les étapes suivantes :
enduction d'une surface d'une couche adhésive (1) avec un adhésif, la surface opposée de la couche adhésive (1) comprenant deux régions de surface, la première région (11) et la deuxième région (12), la couche adhésive (1) étant la couche inférieure du timbre transdermique et étant faite d'une matière flexible ayant une bonne perméabilité ;
fixation d'une couche médicamenteuse (2), d'un transducteur à ultrasons (3) et d'une unité d'excitation (4) sur la première région (11), l'unité d'excitation (4) comportant une alimentation électrique (41) et un circuit d'excitation à ultrasons (42) qui se trouve sur la surface arrière du transducteur à ultrasons (3), l'alimentation électrique (41) étant connectée électroniquement au circuit d'excitation à ultrasons (42) et au transducteur à ultrasons (3) ;
enduction de la deuxième région (12) avec un adhésif et combinaison de celle-ci avec la couche de couverture (5) ;
pulvérisation d'un médicament sur une matière absorbante ou imprégnation d'une matière absorbante avec un médicament de façon à former une couche médicamenteuse (2) ;
fixation de la couche médicamenteuse (2) sur la première région (11), avec la surface émettant les ondes ultrasonores du transducteur à ultrasons (3) attachée directement à la couche médicamenteuse (2) ;
couverture complète de la couche médicamenteuse (2), du transducteur à ultrasons (3) et de l'unité d'excitation (4) et fixation de ceux-ci sur la première région (11) de la couche adhésive (1) avec une partie de la couche de couverture (5), et combinaison de l'autre partie avec la deuxième région (12) de la couche adhésive (5) pour former un bord de collage flexible à volonté de telle façon que le timbre transdermique puisse s'adapter sur une surface de peau non plane d'un corps humain ;
mise en place d'un interrupteur à film (7) pour garder l'alimentation électrique (41) inactive, une extrémité de l'interrupteur à film (7) étant exposée à l'extérieur du timbre transdermique afin de pouvoir être retirée en fonctionnement pour activer l'alimentation électrique (41).

9. Procédé selon la revendication 8, dans lequel la première région (11) ne se trouve pas au centre de la couche adhésive (1) et la deuxième région (12) est une périphérie asymétrique.
